# EUROPÄISCHE PATENTSCHRIFT

(11) **EP 1 066 255 B1**
(45) Veröffentlichungstag und Bekanntmachung des Hinweises auf die Patenterteilung: **11.08.2004**
(21) Anmeldenummer: 99913237.6
(22) Anmeldetag: 08.03.1999
(51) Int. Cl.: C07D 201/08

(54) **VERFAHREN ZUR HERSTELLUNG VON LACTAMEN MIT HILFE VON OLIGOPHOSPHAT-KATALYSATOREN**
METHOD FOR PRODUCING LACTAMS USING OLIGOPHOSPHATE CATALYSTS
PROCEDE DE PRODUCTION DE LACTAMES EN PRESENCE D'UN OLIGOPHOSPHATE COMME CATALYSEUR

(30) Priorität: 18.03.1998 DE 19811880
(43) Veröffentlichungstag der Anmeldung: 10.01.2001
(73) Patentinhaber: BASF AKTIENGESELLSCHAFT, 67056 Ludwigshafen (DE)
(72) Erfinder: EIERMANN, Matthias, D-67117 Limburgerhof (DE); ANSMANN, Andreas, D-69168 Wiesloch (DE); FLICK, Klemens, D-76863 Herxheim (DE)
(74) Vertreter: Isenbruck, Günter, Dr.
(86) Internationale Anmeldenummer: PCT/EP1999/001463
(87) Internationale Veröffentlichungsnummer: WO 1999/047500

(56) Entgegenhaltungen:
- EP-A- 0 659 741
- WO-A-95/14665
- WO-A-96/16936
- WO-A-96/17826
- WO-A-96/22974
- WO-A-99/28296
- DE-A- 4 319 134
- DE-A- 4 339 648
- US-A- 2 357 484

## Beschreibung

Lactame sind vielseitig einsetzbare Verbindungen. So ist das N-Methylbutyro lactam (N-Methylpyrrolidon) ein gängiges Lösungsmittel und ȧ-Caprolactam ein bedeutendes Monomer für die Herstellung von Polyamidfasern.

Lactame können durch cyclisierende Hydrolyse von Aminonitrilen in der Gasphase hergestellt werden. Dabei kommen Katalysatoren mit dehydratisierenden Eigenschaften, wie Aluminiumoxid, Silicagel oder Borphosphorsäure zum Einsatz.

In EP-A 0 659 741 ist die Herstellung von Lactamen aus Aminonitrilen und Wasser durch cyclisierende Hydrolyse in der Gasphase beschrieben, wobei als Katalysatoren Metallorthophosphate, insbesondere Aluminium-, Zirkon-, Niob- und Lanthanorthophosphate, eingesetzt werden. Die Katalysatoren können zusätzlich mit basischen Alkali- bzw. Erdalkaliverbindungen, bevorzugt von Caesium, Rubidium und Kalium, imprägniert sein.

Die Selektivität der nach dem Stand der Technik eingesetzten Katalysatoren läßt jedoch noch zu wünschen übrig. Die Bildung von Nebenprodukten erschwert einerseits die Isolierung der Lactame und kann andererseits zu Vergiftungen der eingesetzten Katalysatoren führen.

Aufgabe der vorliegenden Erfindung ist es, einen Katalysator zur Herstellung von Lactamen durch cyclisierende Hydrolyse von Aminonitrilen bereitzustellen, der sich bei hohen Umsätzen durch eine hohe Selektivität auszeichnet.

Gelöst wird die Aufgabe durch ein Verfahren zur Herstellung von Lactamen durch cyclisierende Hydrolyse von Aminonitrilen in der Gasphase in Gegenwart eines Metallphosphat-Katalysators, das dadurch gekennzeichnet ist, daß als Katalysator ein oder mehrere Oligophosphate der allgemeinen Formel (I)

M(POₐ)_{b} (I)

worin M für Lanthan oder Cer steht, a > 2,6 und < 3,5 ist und b so gewählt ist, daß Ladungsneutralität herrscht,
oder ein Gemisch von einem oder mehreren Oligophosphaten der allgemeinen Formel (I) mit einem oder mehreren weiteren Salzen eines Metalls der Gruppe 3 oder 4 des Periodensystems einschließlich der Lanthaniden mit einer anorganischen Säure, wobei das Molverhältnis der weiteren Salze zu den Oligophosphaten der allgeneinen Formel (I) bis zu 5:1 beträgt, eingesetzt werden.

Als Katalysator können ein oder mehrere Oligophosphate der allgemeinen Formel (I) eingesetzt werden. Die Formel (I) ist als Verhältnisformel aufzufassen und nicht als Summenformel tatsächlich existierender Verbindungen. Oligophosphate im Sinne der vorliegenden Erfindung sind alle Phosphate, die sich formal von Säuren ableiten, welche durch Kondensation von Orthophosphorsäure unter Wasseraustritt erhältlich sind. Durch Kondensation von Orthophosphorsäure H₃PO₄ werden unter intermolekularer Wasserabspaltung kettenförmige Oligophosphorsäuren Hₙ₊₂PₙO₃ₙ₊₁ (Tri-, Tetra-, Pentaphosphorsäure usw.; n = 3, 4, 5, usw.) oder (für große n) polymere Polyphosphorsäuren erhalten. Ab der Triphosphorsäure ist neben einer inter- auch eine intramolekulare Kondensation unter Bildung ringförmiger Metaphosphorsäuren HₙPₙO₃ₙ (Tri-, Tetrametaphosphorsäure usw; n = 3, 4, usw.), andererseits neben einer kettenverlängernden auch eine kettenverzweigende Kondensation unter Bildung verzweigter Ultraphosphorsäuren (z.Bsp. Isotetraphosphorsäure H₆P₄O₁₃) möglich. Formales Endprodukt der Kondensation ist polymeres Phosphorpentaoxid P₂O₅. Für die von diesen Säuren abgeleiteten Oligophosphate der allgemeinen Formel (I) gilt, daß der Wert von a zwischen dem entsprechenden Wert für polymeres Phosphorpentaoxid (2,5) und dem für Orthophosphat (4,0) liegt. Es gilt also 2,6< a < 3,5. Bevorzugt beträgt der Wert von a 3 bis 3,5. Insbesondere ist a = 3.

b ist so gewählt, daß Ladungsneutralität herrscht. Liegt in den Oligophosphaten Phosphor ausschließlich als fünfwertiger Phosphor vor, so gilt insbesondere b = (2a - 5)/z, wobei z die Ladungszahl der M-Kationen ist.

M steht für La und Ce, ganz besonders bevorzugt für La. Die erfindungsgemäß eingesetzten Oligophosphate können eine oder mehrere Arten von Metallen M enthalten, bevorzugt enthalten sie nur eine einzige Art von Metallen M.

Als Katalysator kann ein bestimmtes Oligophosphat, bevorzugt Trimetaphosphat, oder ein Gemisch aus mehreren verschiedenen Oligophosphaten der allgemeinen Formel (I) eingesetzt werden.

Als Katalysator besonders bevorzugt ist Trimetaphosphat, insbesondere Lanthantrimetaphosphat (LaP₃O₉).

Als Katalysatoren können ferner Gemische von einem oder mehreren der vorstehend genannten Oligophosphate der allgemeinen Formel (I) mit einem oder mehreren weiteren Salzen der Metalle der Gruppe 3 oder 4 des Periodensystems einschließlich der Lanthaniden, also für Sc, Y, Ti, Zr, Hf, La, Ce, Pr, Nd, Sm, Eu, Gd, Tb, Dy, Ho, Er, Tm, Yb und Lu, bevorzugt für die Lanthaniden, besonders bevorzugt für La und Ce, gant besonders bevorzugt für La, mit anorganischen Säuren eingesetzt werden. Die in den Oligophosphaten und in den weiteren Salzen vorliegenden Metalle können gleich oder verschieden sein, bevorzugt sind sie gleich. Bevorzugte weitere Salze sind die Orthophosphate, Sulfate, Carbonate, Silikate, Arsenite, Arsenate, Antimonite, Antimonate und Nitrate, besonders bevorzugt die Orthophosphate der genannten Metalle.

Die Oligophosphate der allgemeinen Formel (I) können allein oder im Gemisch mit den weiteren Salzen eingesetzt werden. Im allgemeinen beträgt das Verhältnis weiterer Salze zu Oligophosphaten bis zu 5:1, ganz besonders bevorzugt von 0,1:1 bis 5:1 und insbesondere von 1:1 bis 5:1.

Als Katalysator weiterhin besonders bevorzugt ist ein Gemisch aus Trimetaphosphat und Orthophosphat, insbesondere mit Lanthan als Metall M.

Die Oligophosphate der allgemeinen Formel (I) und die weiteren Salze können jeweils pro Formeleinheit bis zu 5 Moleküle Wasser enthalten.

Die Herstellung des Katalysators erfolgt im allgemeinen aus dem Nitrat, Nitrit, Carbonat, Formiat, Acetat, Oxalat oder einem anderen Salz einer organischen Säure, bevorzugt aber aus dem Nitrat des Metalls M und Ammoniumphosphat bevorzugt ist, als Precursoren. Diese Komponenten werden fein pulverisiert im gewünschten molaren Verhältnis miteinander inniglich vermischt. Das gewählte molare Verhältnis Phosphor:Metall (= b) findet sich nach erfolgter Reaktion im Produkt, dem Oligophosphat oder dem Gemisch aus verschiedenen Oligophosphaten, wieder. Nach dem Vermischen der Precursoren werden diese, beispielsweise im offenen Tiegel, zur Zersetzung der Precursoren für im allgemeinen 2 bis 48 Stunden, bevorzugt 8 bis 36 Stunden, auf Temperaturen von im allgemeinen 140 bis 200°C, bevorzugt 150 bis 180°C, langsam erhitzt. Danach wird langsam für 1 bis 8 Tage, bevorzugt für 2 bis 5 Tage auf 250 bis 900°C, bevorzugt 400 bis 650°C erhitzt, um die Umsetzung zum Oligophosphat zu vollenden. Auf diese Weise können beliebige Oligophosphate mit einem Phosphor:Metall Verhältnis ≥ 3 als Gemische, zum Teil aber auch in reiner Form erhalten werden. Beispielsweise ist Trimetaphosphat auf diese Weise in reiner Form zugänglich.

Gegenstand der Erfindung ist auch ein Verfahren zur Herstellung eines Katalysators, mit den Schritten:
a) Herstellen eines Gemisches aus Ammoniumdihydrogenphosphat und dem Nitrat des Metalls M im gewünschten molaren Verhältnis
b) Stufenweises Erhitzen dieses Gemisches zur Zersetzung der Precursoren und Feststoffreaktion zum Metalloligophosphat.

Metaphosphate können ferner erhalten werden, indem M aus einer Lösung seines Salzes mit Pyrophosphorsäure H₄P₂O₇ als Dihydropyrophosphat gefällt wird und der erhaltene Niederschlag zum Metaphosphat calciniert wird.

Gegenstand der Erfindung ist auch ein Verfahren zur Herstellung eines Katalysators mit den Schritten:
a) Herstellen einer Lösung, welche ein Salz des Metalls M enthält;
b) Fällen von M aus dieser Lösung als Dihydropyrophosphat und Abtrennung des Niederschlages, welcher das Metalldihydropyrophosphat enthält;
c) gegebenenfalls Waschen und Trocknen des Niederschlages;
d) Calcinieren des Niederschlages.

Das Fällen von M als Dihydropyrophosphat erfolgt vorzugsweise bei einem pH-Wert von im allgemeinen 0,5 bis 4, bevorzugt 0,8 bis 2. Zur Fällung kann in einer Lösung, welche ein Salz des Metalls M und Pyrophosphorsäure enthält, durch Zugabe von Base ein bestimmter pH-Wert eingestellt werden. Bevorzugte Basen sind Ammoniak, Alkalihydroxide, primäre, sekundäre und tertiäre Amine, besonders bevorzugt ist Ammoniak. Es kann ferner eine Lösung, welche das Salz des Metalls M enthält, zu einer wäßrigen alkalischen Lösung von Pyrophosphorsäure gegeben werden. Geeignete Salze sind wasserlösliche Salze des Metalls M, bevorzugt sind die Nitrate.

Die Konzentration der Metallsalzlösung beträgt im allgemeinen 0,1 bis 1,5 mol/l, bevorzugt 0,8 bis 1,1, die der Pyrophosphorsäure im allgemeinen 0,1 bis 5, bevorzugt 2 bis 4 mol/l.

Das Fällen von M als Pyrophosphat kann in der Kälte oder in der Wärme vorgenommen werden. Zur Herstellung von Katalysatoren, die im wesentlichen aus den Oligophosphaten der allgemeinen Formel (I) bestehen und keine nennenswerten Anteile an Orthophosphaten enthalten, wird vorzugsweise in der Kälte gefällt, wobei besonders bevorzugt unter Eiskühlung gearbeitet wird. Zur Herstellung von Katalysatoren, die als weitere Salze Orthophosphate enthalten, wird vorzugsweise in der Wärme gefällt, wobei die Temperatur im allgemeinen 30 bis 100°C, bevorzugt 60 bis 90°C beträgt.

Der pyrophosphathaltige Niederschlag wird abgetrennt, gegebenenfalls gewaschen und getrocknet, und anschließend calciniert. Das Trocknen erfolgt im allgemeinen bei einer Temperatur von 60 bis 180°C, bevorzugt 100 bis 150°C, das Calcinieren im allgemeinen bei 300 bis 900, bevorzugt bei einer 500 bis 700°C über einen Zeitraum von im allgemeinen 0,5 bis 10 h, bevorzugt 2 bis 4 h.

Gemische von Oligophosphaten der allgemeinen Formel (I) und einem oder mehreren weiteren Salzen können nach einer der folgenden Methoden erhalten werden:
- durch Feststoffreaktion aus einem Nitrat des Metalls M, Ammoniumdihydrogenphosphat und einem Ammoniumsalz der anorganischen Säure, von denen sich die weiteren Salz ableiteten;
- durch Kofällung von Dihydropyrophosphaten des Metalls M und weiteren Salzen und anschließendem Calcinieren;
- durch Eindampfen von Lösungen, enthaltend Phosphorsäure oder Oligophosphorsäuren, ggf. andere anorganische Säuren, sowie das Metall M, welches aus dem entsprechenden Oxid im gewünschten molaren Verhältnis stammt, und gegebenenfalls anschließendem Calcinieren.

Die Katalysatormaterialien können in beliebiger Form, z.B. als Pulver, als Splitt oder als Formkörper eingesetzt werden. Als Formkörper werden die Katalysatormaterialien beispielsweise in Form von Strängen oder Kugeln eingesetzt. Zur Formgebung kann ein Binder zugesetzt werden, beispielweise Aerosil, Kartoffelstärke oder Celluloseether (z.B. Walocel®). Die Katalysatormaterialien können ferner auf einem Träger, wie Tonerde, Kieselgel, Kohle, Siliciumcarbid oder Siliciumnitrid, aufgebracht sein.

Bevorzugt wird der Katalysator in Form von Splitt oder Formkörpern eingesetzt. Der Katalysatorschüttung können zusätzliche Komponenten zur Selektivitätssteigerung in einer Menge von bis zu 70 Vol% beigemischt werden. Beispiele sind Siliciumdioxid, Siliciumnitrit und Siliciumcarbid, bevorzugt Siliciumdioxid, besonders bevorzugt Quarz.

In dem erfindungsgemäßen Verfahren einsetzbarer Aminonitrile sind aliphatische Aminonitrile mit wenigstens zwei, bevorzugt 3 bis 20 Atomen, in der Kette zwischen der Amino- und der Nitrilgruppe. In der Regel sind dies Kohlenstoffatome, es können aber auch ein oder mehrere, vorzugsweise nicht mehr als drei, Bor-, Stickstoff-, Phosphor-, Sauerstoff- und/oder Schwefelatome in nicht benachbarter, sonst aber beliebiger Position innerhalb der Kette enthalten sein. Die Aminogruppe kann einfach mit einer geradkettigen oder verzweigten Alkylgruppe mit bis zu 20 Kohlenstoffatomen, beispielsweise mit Methyl, Ethyl, n-Propyl, isoPropyl, n-Butyl, sek -Butyl oder tert.-Butyl substituiert sein Vorzugsweise werden in dem erfindungsgemäßen Verfahren Aminonitrile mit 3, 4, 5 oder 6 Kohlenstoffatomen in der Kette zwischen Amino- und Nitrilgruppe ohne Fremdatome, besonders bevorzugt mit primären Aminogruppen, wie 4-Aminobutyronitril, 5-Aminovaleronitril, 6-Aminocapronitril und 7-Aminooenanthonitril, ganz besonders bevorzugt 6-Aminocapronitril, eingesetzt.

Die cyclisierende Hydrierung kann an einem bewegten oder an einem stationären Katalysatorbett durchgeführt werden. Bevorzugt wird die Reaktion an einem stationären Katalysatorbett (Festbett) durchgeführt. Das Festbett kann beispielsweise als einfache Schüttung oder aufgeteilt in mehreren Horden angeordnet sein. Das Festbett kann außerdem in einem oder in mehreren Reaktionsräumen, z.B. in einem Rohrbündelreaktor, angeordnet sein. Das Molverhältnis von Wasser zu Aminonitril beträgt im allgemeinen 1 bis 50, bevorzugt 1 bis 15. Die Reaktionstemperatur beträgt im allgemeinen 200° bis 550°C, bevorzugt 300° bis 400°C. Bei Temperaturen unterhalb 200°C ist das Verdampfen des Aminonitrils erschwert, weiterhin sind hohe Umsätze nur schwer erreichbar. Bei Temperaturen oberhalb 550°C treten vermehrt Neben- und Zersetzungsprodukte auf.

Die Reaktion wird im allgemeinen bei einem Druck von 0,01 bis 10 bar, bevorzugt bei Normaldruck durchgeführt.

Die Reaktion kann in Gegenwart eines Inertgases, beispielweise Argon oder Stickstoff, durchgeführt werden, wobei das Inertgas in einem bis zu 100-fachen Überschuß über das Aminonitril vorliegen kann.

Der Reaktoraustrag enthält in der Regel neben dem Lactam als Produkt unumgesetztes Aminonitril und Wasser sowie Ammoniak bzw. Amine und in geringen Mengen Nebenprodukte wie Aminocarbonsäureamide. Das Lactam kann aus dem Reaktionsaustrag durch übliche Trennverfahren wie Destillation, Extraktion oder Kristallisation gewonnen werden.

Die Katalysatorbeladung beträgt typischerweise 50 bis 2000 g, bevorzugt 500 bis 2000 g, des Aminonitrils pro Liter Katalysator und Stunde. Die Umsätze betragen, bezogen auf Aminonitril, 70 bis 99,9%. Die Selektivität der Lactambildung liegt im allgemeinen oberhalb 85%, bevorzugt oberhalb 90%, besonders bevorzugt oberhalb 93%, bezogen auf eingesetztes Aminonitril. Selektivitäten oberhalb 95% sind möglich. Diese Selektivitätswerte werden auch nach Betriebszeiten des Katalysators von mehreren 100 Stunden noch erreicht.

Die Erfindung wird durch die nachstehenden Beispiele zusätzlich näher erläutert.

### Katalysatorpräparation

### Katalysator 1:

Im molaren Verhältnis von 3:1 werden fein zerriebene Pulver von (NH₄)H₂PO₄ und La(NO₃)₃•6H₂O als Precursor innig miteinander in einem Porzellantiegel vermischt. Zur Zersetzung der Precursor wird die Mischung für 24 h bei 150°C und anschließend für 12 h bei 180°C gehalten und anschließend langsam auf 600°C aufgeheizt. Nach vier Tagen kühlt man ab und erhält so eine feste Schmelze aus LaP₃O₉, die sich leicht zerkleinern und zu Splitt der Körnung 0,1 bis 0,5 mm verarbeiten läßt.

### Katalysator 2:

Es werden eine Lösung von 0,5 Mol La(NO₃)₃•6H₂O in 500 ml Wasser und von 1,5 Mol Pyrophorsäure in 500 ml Wasser hergestellt. Die erste Lösung wird zu der zweiten unter Rühren zugetropft. Unter Kühlung mit Eis werden anschließend 3 Mol einer mit Wasser im Verhältnis 1:1 verdünnten wäßrigen konzentrierten NH₃-Lösung zugetropft, wobei ein Niederschlag ausfällt. Dieser Niederschlag wird abgetrennt, mit kalter NH₃-Lösung gewaschen und danach 18 h lang bei 150°C getrocknet. Die so erhaltene Masse wird zerkleinert und zu Splitt der Körnung 0,1 bis 0,5 mm verarbeitet. Danach wird für 9,5 h bei 380°C und anschließend für 2 h bei 550°C die Masse zum Methaphosphat zersetzt.

### Katalysator 3:

Es werden eine Lösung von 0,8 Mol La(NO₃)₃•6H₂O in 736 ml Wasser und eine Lösung von 0,92 Mol Pyrophosphorsäure in 800 ml Wasser hergestellt. Die Pyrophosphorsäure wird mit konzentrierter NH₃-Lösung auf einen pH-Wert von 10,0 eingestellt und anschließend wird die Metallsalzlösung langsam zugetropft. Der entstehende Niederschlag wird noch 1,5 h bei 80°C gerührt. Der Niederschlag wird abzentrifugiert, zweimal mit ammoniakalischem Wasser vom pH-Wert 10,0 aufgeschlämmt und wieder abzentrifugiert. Die so erhaltende Masse wird für 12 h bei 110°C getrocknet und danach zu Splitt der Körnung 0,1 bis 0,5 mm verarbeitet. Anschließend wird 4 h lang bei 700°C calciniert, wobei eine Mischung aus Meta- und Orthophosphat erhalten wird.

### Katalysator V (Vergleichsbeispiel):

Gemäß EP-A 0 659 741 wird ein Katalysator hergestellt, der aus reinem Lanthanorthophosphat besteht. Dazu werden eine Lösung von 1,0 Mol La(NO₃)₃•6H₂O in 3000 ml Wasser und eine Lösung von 2,0 Mol (NH₄)₂HPO₄ in 1500 ml Wasser hergestellt. Die zweite Lösung wird zu der ersten bei Raumtemperatur langsam unter Rühren zugetropft, wobei ein Niederschlag fällt. Danach wird der pH-Wert der Suspension mit wäßriger NH₃-Lösung auf 6,0 eingestellt. Nach 30 minütigem Rühren wird der Niederschlag auf einer Nutsche mit 24 l Wasser gewaschen und danach für 12 h bei 120°c getrocknet. Die erhaltene Masse läßt sich gut zerkleinern und wird zu Splitt der Körnung 0,1 bis 0,5 mm verarbeitet. Schließlich wird der Splitt bei 500°C während 4 h calciniert. Das Calcinierungsprodukt besteht gemäß seinem XRD-Spektrum aus reinem Lanthanorthophosphat.

### Cyclisierungsexperiment

Die oben beschriebenen Katalysatoren werden in einem elektrisch beheizten Rohrreaktor mit einem inneren Durchmesser von 30 mm, der von unten beginnend mit 20 ml Quarzsplitt, darauf 20 ml Katalysator als Splitt < 0,1 mm und darauf 50 ml Quarzsplitt als Verdampferzone beschickt ist, getestet. Nach Befüllen des Reaktors mit Katalysator wird dieser, wie in EP-A 0 659 741 beschrieben, im Luftstrom auf 400°C erhitzt und anschließend unter Stickstoff auf die Reaktionstemperatur abgekühlt.

Der Reaktor wird in Abwärtsfahrweise betrieben. 6-Aminocapronitril wird mit 750 g je l Katalysator und Stunde als 50 gew.-%ige wäßrige Lösung aufgegeben. Die Reaktion erfolgt bei 360°C unter Zusatz von 10 l/h Stickstoff als Trägergas bei Normaldruck. Der Umsatz an 6-Aminocapronitril (ACN) und die Selektivität für Caprolactam (CPL) werden mittels Gaschromatographie mit internem Standard und über die Massenbilanz bestimmt. Zur exakten quantitativen Bestimmung von Selektivität und Umsatz werden Proben über mehrere Stunden akkumuliert. Die Ergebnisse sind in Tabelle 1 zusammengestellt.

**Tabelle 1**

| Katalysator | Selektivität CPL | Umsatz ACN | Zeitpunkt der Messung |
|---|---|---|---|
| 1 | 93,5% | 99,2% | 53 h |
| | 91,7% | 99,2% | 124 h |
| 2 | 92,4% | 99,3% | 165 h |
| | 92,9% | 99,3% | 555 h |
| 3 | 98,4% | 99,6% | 276 h |
| | 97,0% | 99,6% | 494 h |
| V | 85,8% | 99,7% | 70 h |
| | 88,5% | 99,3% | 166 h |

Die Bestimmungen wurden vorgenommen, nachdem die Reaktion mindestens zwei Tage lang stabil gelaufen war. Die Analyse des gebrauchten Katalysators V nach 166 h ergab, daß es sich weiterhin um reines Lanthanorthophosphat handelte.

Die Beispiele zeigen, daß durch die erfindungsgemäß eingesetzten oligophosphathaltigen Katalysatoren 1 bis 3 höhere Umsätze und Selektivitäten bezüglich der Caprolactambildung erreicht werden als mit reinem Ortholanthanphosphat (Katalysator V).

## Patentansprüche

1. Verfahren zur Herstellung von Lactamen durch cyclisierende Hydrolyse von Aminonitrilen in der Gasphase in Gegenwart eines Metallphosphat-Katalysators, **dadurch gekennzeichnet, daß** als Katalysator ein oder mehrere Oligophosphate der allgemeinen Formel (I)
M(POₐ)_{b} (I)
worin M für Lanthan oder Cer steht, a von 2,6 bis 3,5 beträgt und b so gewählt ist, daß Ladungsneutralität herrscht,
oder ein Gemisch von einem oder mehreren Oligophosphaten der allgemeinen Formel (I) mit einem oder mehreren weiteren Salzen eines Metalls der Gruppe 3 oder 4 des Periodensystems einschließlich der Lanthaniden mit einer anorganischen Säure, wobei das Molverhältnis der weiteren Salze zu den Oligophosphaten der allgemeinen Formel (I) bis zu 5 : 1 beträgt, eingesetzt werden.

2. Verfahren nach Anspruch 1, **dadurch gekennzeichnet, daß** die weiteren Salze ausgewählt sind aus der Gruppe bestehend aus Orthophosphat, Sulfat, Carbonat, Silikat, Arsenit, Arsenat, Antimonit, Antimonat und Nitrat.

3. Verfahren nach Anspruch 1 oder 2, **dadurch gekennzeichnet, daß** das Oligophosphat Trimetaphosphat ist.

4. Verfahren nach einem der Ansprüche 1 bis 3, **dadurch gekennzeichnet, daß** das Molverhältnis der weiteren Salze zu den Oligophosphaten der allgemeinen Formel (I) 1 bis 5 beträgt.

5. Verfahren nach einem der Ansprüche 1 bis 4, **dadurch gekennzeichnet, daß** das weitere Salz Orthophosphat ist.

6. Verfahren nach einem der Ansprüche 1 bis 5, **dadurch gekennzeichnet, daß** als Aminonitrile 4-Aminobutyronitril, 5-Aminovaleronitril, 6-Aminocapronitril und 7-Aminonanthonitril eingesetzt werden.

7. Verfahren zur Herstellung eines Katalysators, wie er in Anspruch 1 beschrieben ist, mit den Schritten:
a) Herstellen einer Lösung, welche ein Salz des Metalls M und enthält;
b) Fällen des Metalls M aus dieser Lösung als Dihydropyrophosphat und Abtrennung des Niederschlages, welcher das Metalldihydropyrophosphat enthält;
c) gegebenenfalls Waschen und Trocknen des Niederschlags;
d) Calcinieren des Niederschlages;

8. Verfahren zur Herstellung eines Katalysators, wie er in Anspruch 1 beschrieben ist, mit den Schritten:
a) Herstellen eines Gemisches aus Ammoniumdihydrogenphosphat und dem Nitrat des Metalls M im gewünschten molaren Verhältnis
b) Stufenweises Erhitzen dieses Gemisches zur Zersetzung der Precursoren und Feststoffreaktion zum Metalloligophosphat.

9. Verwendung von Metalloligophosphaten oder von Gemischen von Metalloligophosphaten und Metallsalzen von Metallen der Gruppen 3 und 4 des Periodensystems einschließlich der Lanthaniden, wie sie in einem der Ansprüche 1 bis 6 definiert sind, als Katalysatoren für die cyclisierende Hydrolyse von Aminonitrilen zu Lactamen.

10. Verwendung nach Anspruch 9 zur Herstellung von Caprolactam.

## Claims

1. A process for preparing lactams by hydrolytic cyclization of aminonitriles in the gas phase in the presence of a metal phosphate catalyst, **characterized in that** it comprises using a catalyst comprising one or more oligophosphates of the general formula (I)
M(POₐ)_{b} (I)
where M is lanthanum or cerium, a is from 2.6 to 3.5, and b is such that electrical neutrality is ensured,
or a mixture of one or more oligophosphates of the general formula (I) with one or more further salts of a metal of group 3 or 4 of the periodic table, including the lanthanides, with an inorganic acid, wherein the molar ratio of said further salts to said oligophosphates of the general formula (I) is up to 5:1.

2. A process as claimed in claim 1, **characterized in that** said further salts are selected from the group consisting of orthophosphate, sulfate, carbonate, silicate, arsenite, arsenate, antimonite, antimonate and nitrate.

3. A process as claimed in claim 1 or 2, **characterized in that** said oligophosphate is trimetaphosphate.

4. A process as claimed in any of claims 1 to 3, **characterized in that** the molar ratio of said further salts to said oligophosphates of the general formula (I) is within the range from 1 to 5.

5. A process as claimed in any of claims 1 to 4, **characterized in that** said further salt is orthophosphate.

6. A process as claimed in any of claims 1 to 5, **characterized in that** said aminonitriles are 4-aminobutyronitrile, 5-aminovaleronitrile, 6-aminocapronitrile and 7-aminoenanthonitrile.

7. A process for preparing a catalyst as described in claim 1, which comprises the steps of:
a) preparing a solution comprising a salt of said metal M;
b) precipitating said metal M from this solution as dihydropyrophosphate and removing the precipitate comprising the metal dihydropyrophosphate;
c) optionally washing and drying said precipitate;
d) calcining said precipitate.

8. A process for preparing a catalyst as described in claim 1, which comprises the steps of :
a) preparing a mixture of ammonium dihydrogenphosphate and the nitrate of said metal M in the desired molar ratio;
b) heating this mixture in stages to decompose the precursors and form the metal oligophosphate in a solid state reaction.

9. The use of metal oligophosphates or mixtures of metal oligophosphates and metal salts of metals of groups 3 and 4 of the periodic table, including the lanthanides, as defined in any of claims 1 to 6, as catalysts for the hydrolytic cyclization of aminonitriles to lactams.

10. A use as claimed in claim 9 for producing caprolactam.

## Revendications

1. Procédé de préparation de lactames par hydrolyse cyclisante d'aminonitriles en phase gazeuse, en présence d'un catalyseur de phosphate métallique, **caractérisé en ce que** l'on met en oeuvre en tant que catalyseur un ou plusieurs oligophosphates de la formule générale (I)
M(POₐ)_{b} (I)
dans laquelle M représente du lanthane ou du cérium, a possède une valeur de 2,6 à 3,5 et b est choisi de manière qu'il règne une neutralité de charge,
ou un mélange d'un ou plusieurs oligophosphates de la formule générale (I) avec un ou plusieurs autres sels d'un métal du groupe 3 ou 4 du système périodique, y compris les lanthanides, avec un acide inorganique, la proportion molaire des autres sels aux oligophosphates de la formule générale (I) allant jusqu'à 5 : 1.

2. Procédé selon la revendication 1, **caractérisé en ce que** les autres sels sont choisis dans le groupe formé par un orthophosphate, un sulfate, un carbonate, un silicate, un arsénite, un arséniate, un antimonite, un antimoniate et un nitrate.

3. Procédé selon la revendication 1 ou 2, **caractérisé en ce que** l'oligophosphate est un trimétaphosphate.

4. Procédé selon l'une quelconque des revendications 1 à 3, **caractérisé en ce que** la proportion molaire des autres sels aux oligophosphates de la formule générale (I) est de 1 à 5.

5. Procédé selon l'une quelconque des revendications 1 à 4, **caractérisé en ce que** l'autre sel est un orthophosphate.

6. Procédé selon l'une quelconque des revendications 1 à 5, **caractérisé en ce que** l'on met en oeuvre comme aminonitrile du 4-aminobutyronitrile, du 5-aminovaléronitrile, du 6-aminocapronitrile et du 7-aminonanthonitrile.

7. Procédé de préparation d'un catalyseur tel que décrit à la revendication 1, comprenant les étapes suivantes:
a) préparation d'une solution contenant un sel du métal M,
b) précipitation du métal M de cette solution sous forme de dihydropyrophosphate et séparation du précipité, lequel contient le dihydropyrophosphate métallique,
c) éventuellement lavage et séchage du précipité,
d) calcination du précipité.

8. Procédé de préparation d'un catalyseur tel que décrit à la revendication 1, comprenant les étapes suivantes:
a) préparation d'un mélange de dihydrogénophosphate d'ammonium et du nitrate du métal M dans la proportion molaire souhaitée,
b) chauffage progressif de ce mélange aux fins de décomposition des précurseurs et de réaction en phase solide pour former l'oligophosphate métallique.

9. Utilisation d'oligophosphates métalliques ou de mélanges d'oligophosphates métalliques et de sels de métaux des groupes 3 et 4 du système périodique, y compris les lanthanides, tels que décrits dans l'une quelconque des revendications 1 à 6, comme catalyseurs pour l'hydrolyse cyclisante d'aminonitriles en lactames.

10. Utilisation selon la revendication 9 pour la préparation de caprolactame.
